# FASCICULE DE BREVET EUROPEEN

(11) **EP 0 427 603 B1**
(45) Date de publication et mention de la délivrance du brevet: **26.05.1999**
(21) Numéro de dépôt: 90403116.8
(22) Date de dépôt: 05.11.1990
(51) Int. Cl.: C07B 39/00, C07C 25/13, C07C 43/225

(54) **Procédé de préparation de dérivés aromatiques fluorés et catalyseurs à base d'acide de lewis**
Verfahren zur Herstellung von fluorierten aromatischen Verbindungen und auf Lewis-Säuren basierte Katalysatoren
Process for the preparation of fluorinated aromatic compounds and catalysts based on Lewis-acids

(30) Priorité: 06.11.1989 FR 8914528; 06.11.1989 FR 8914529; 06.11.1989 FR 8914532
(43) Date de publication de la demande: 15.05.1991
(73) Titulaire: RHODIA CHIMIE, 92408 Courbevoie Cédex (FR)
(72) Inventeur: Garcia, Hervé, F-69190 Saint Fons (FR); Gilbert, Laurent, F-69006 Lyon (FR); Ratton, Serge, F-78100 Saint Germain en Laye (FR); Rochin, Christophe, F-69006 Lyon (FR)
(74) Mandataire: Ricalens, François

(56) Documents cités:
- EP-A- 118 241
- FR-A- 2 134 381
- JP-A- 2 095 438
- US-A- 4 814 524
- JOURNAL OF FLUORINE CHEMISTRY, vol. 27, 1985, Elsevier Sequoia, Lausanne (CH); D.P. ASHTON et al., pp. 263-274

## Description

La présente invention concerne un procédé de préparation de dérivés aromatiques fluorés. Elle concerne plus particulièrement la préparation de dérivés aromatiques qui portent un fluor directement attaché sur le noyau aromatique. Elle concerne encore plus précisément la préparation de fluorobenzènes.

Il est connu depuis déjà très longtemps par exemple selon le brevet US 4075252 de préparer le fluorobenzène à partir des anilines par une réaction de diazotation ou par passage par un intermédiaire triazène puis décomposition du sel de diazonium ou de triazène dans l'acide fluorhydrique. Ces techniques anciennes imposent toujours un procédé en deux étapes, éventuellement réalisées en continu, par exemple selon le brevet EP 205019, qui comporte toujours une étape de formation du sel de diazonium et une étape de décomposition.

Les amines de départ sont des composés onéreux dont il est impossible de se passer pour un tel procédé, ce qui grève considérablement le prix d'obtention final du fluorobenzène.

Il est aussi connu selon le brevet anglais n° 1582427 de préparer le fluorobenzène par mise en contact à environ 200°C d'un phénol avec une solution d'acide fluorhydrique contenant un acide de Lewis en quantité pondérale comprise entre 0,5 et 25 % calculée par rapport à l'acide fluorhydrique introduit.

Les rendements obtenus en fluorobenzène sont toujours inférieurs à 25% pour des durées de réactions de 6 heures.

Il est encore décrit dans le brevet européen n°118241 un procédé de préparation du fluorobenzène par passage d'un ester de l'acide fluoroformique sur alumine platinée à une température comprise entre 200 et 600°C. Ce procédé réalisé en phase gazeuse avec des rendements annoncés en fluorobenzène atteignant 60 %, a fait l'objet d'une publication ultérieure plus complète (The preparation of fluoroarenes by the catalytic decarboxylation of aryl fluoroformates, Journal of Fluorine Chemistry, 27 (1985) 263 274, David P. ASHTON, T. ANTHONY RYAN, BRIAN R. WEBSTER and BRETT A. WOLFINDALE), et souffre de nombreux inconvénients parmi lesquels le coût du réactif et la production de sous produits en nombre et en quantité importants.

Qui plus est, le catalyseur s'empoisonne très vite et la régénération subséquente nécéssaire grève beaucoup trop le procédé pour que l'on puisse envisager son utilisation à l'échelle industrielle.
La raison invoquée pour expliquer l'empoisonnement du catalyseur est la dégradation de ce dernier par l'action de l'acide fluorhydrique.

C'est pourquoi, un des buts de la présente invention est de fournir un procédé de préparation du fluorure d'aryle qui permette d'éviter ou d'espacer les cycles de régénération.

Un autre but de la présente invention est de fournir un autre procédé qui utilise des composés de départ peu onéreux.

Ces buts et d'autres qui apparaitront par la suite sont atteints au moyen d'un procédé de préparation de fluorure d'aryle qui comporte une étape réactionnelle de mise en contact d'un mélange gazeux contenant l'acide fluorhydrique et un halogénoformiate ou un carbonate d'aryle (et leurs équivalents) avec un catalyseur à base d'acide de Lewis.

En géneral, pour cette transformation, que l'on peut qualifier de halogènation-décarboxylation, le rapport molaire entre l'acide fluorhydrique et l'halogénoformiate ou le carbonate d'aryle est au moins égal à 1 (avec à la rigueur, lorsque il n'y a pas d'halogène à apporter au substrat, par exemple par échange avec l'acide halohydrique gazeux, la possibilité d'abaisser ce rapport à 10⁻¹), avantageusement compris entre 5 et 1000, de préférence entre 10 et 1000 (sauf quand cela est précisé autrement, les zéros ne sont pas des chiffres significatifs).

Lorsque le substrat comporte plus d'un halogène à échanger, avec l'halogène de l'acide halohydrique, les valeurs inférieures doivent être relevées de 2 fois au moins la quantité stoechiométrique nécessaire à l'échange complet. Ainsi, dans le cas de l'utilisation du trichlorométhoxy benzène, la limite inférieure devient 1 + 6 soit 7 pour le rapport entre l'acide fluorhydrique et le substrat.

Avantageusement, la température est comprise entre 200 et 800° C de préférence entre 200 et 500°C.

Ces paramètres peuvent être optimisés à l'interieur des fourchettes ci-dessus, quoique le simple respect de ces dernières donne de bons résultats. Le mélange gazeux peut comporter, outre les réactifs, un gaz inerte (dans les conditions opératoires) vis-à-vis des espèces réactionnelles et du catalyseur et jouer un rôle de gaz vecteur ou de diluant lorsque les molécules d'halogénoformiate ou de carbonate sont suffisament réactives pour qu'elles puissent réagir sur elle-mêmes ou sur le fluorobenzène produit par la réaction. Le mélange gazeux est avantageusement à une pression totale comprise entre 10⁻² et 20 MPa de préférence de 10⁻¹ à 20, plus usuellement de 10⁻¹ à 2.

Il est souhaitable que le mélange réactionnel soit anhydre, c'est-à-dire que la teneur molaire en eau soit de préférence au plus égale à 10 % de celle en substrat arylique de préférence 1 %. Le caractère anhydre du mélange gazeux réactionnel est limité par celui de l'acide fluorhydrique commercial réputé anhydre.

Les équivalents de la fontion Halogènoformiate ou carbonate sont les composés de formule générale :

-O-CY-A

où Y représente un chalcogène de préférence léger (S ou O) avantageusement oxygène ou bien deux halogènes de préférence semblables ; où A représente, un halogène ou un groupe -OR_{'2} voire un groupe -NR_{'2}R_{"2} dans lequel R_{'2} représente :
un radical aliphatique contenant 1 à 4 atomes de carbone éventuellement halogéné ; ou
un radical aromatique (éventuellement substitué par un groupe alkyle contenant 1 à 4 atomes de carbone, alcoxy contenant 1 à 4 atomes de carbone, nitro, halogéno, hydroxy) ;
dans lequel R_{"2}
représente un hydrogène ;
un radical aliphatique contenant 1 à 4 atomes de carbone éventuellement halogéné ou ;
un radical aromatique (éventuellement substitué par un groupe alkyle contenant 1 à 4 atomes de carbone, alcoxy contenant 1 à 4 atomes de carbone, nitro, halogéno, hydroxy) ;

Sous le concept d'acide de Lewis, il faut entendre les composés accepteurs de doublet électronique. Les paradigmes de ces composés sont à trouver dans l'ouvrage édité par G.A. OLAH "Friedel-Crafts and related réactions" 1963 tome I,pages 191 à 197, 201, 202 et 225 à 291.

Le catalyseur à base d'acide de Lewis contient avantageusement une espèce chimique choisie dans le groupe constitué par les éléments de transition (éléments à sous couche d en cours de remplissage de IV A à II B), les éléments métalliques des colonnes III A et B, IV B, V B, (pour ces deux dernières colonnes avantageusement à partir de la troisième période, de préférence de la quatrième) y compris le Germanium, voire le silicium et les alcalino-terreux de rang bas (au plus le magnésium) sous forme de chalcogénure(s), de préférence oxyde(s), d'halogénure(s), de carbonate(s), sulfate(s), phosphate(s) et des sels organique(s) décomposable(s), ainsi que par leurs mélanges ; les oxy-halogénures font notamment partie dudit groupe.

D'une manière générale conviennent bien tout dérivé ou mélange qui soumis au mélange réactionnel donnent au moins partiellement des fluorures y compris des chalcogéno-fluorures, lesquels peuvent être mixte et comporter d'autre élements, dès lors que le caractère d'acide de Lewis est préservé.

Ainsi les fluorures et les oxyfluorures et les espèces qui le deviennent au moins partiellement sous l'action de l'acide fluorhydrique gazeux, donnent des résultats particulièrement satisfaisants.

En ce qui concerne les acides de Lewis dérivant des éléments de la colonne III A, les éléments les plus visés sont les terres rares y compris le scandium ainsi que l'actinium, le thorium et l'uranium.

Parmi les éléments de transition, on peut citer ceux dont le symbole suit : Cr, Fe, Mo, W, Ag, Cu, Zn, Ti, Co, Ni, Ta, Nb, V, Au, Re.

Les éléments de transition préférés sont le chrome, le nickel, le cobalt, le fer et/ou le molybdène.

Les éléments préférés sont le chrome, le fer, l'étain, le germanium, le plomb, les éléments de la colonne III B (de préférence Al, Ga, In) et l'antimoine. Il est fait référence à la classification Française (cf.Bull. Soc. Chim. n°1 Janvier 1966).

Ladite espèce chimique peut être un alliage métallique corrodable pour donner un acide de Lewis en surface. Ainsi elle peut être un alliage métallique notamment des aciers de préférence austénitiques tels que les aciers contenant de préférence chrome, nickel et éventuellement du molybdène. L'espèce chimique peut également être un acier du type Hostalloy C 276. Dans ce cas, les taux de conversion volumiques sont faibles.

Ladite espèce chimique peut être une zéolithe, de préférence de type Y, c'est-à-dire avec un taux Si/Al très faible.

Il est possible; voire avantageux, de soumettre avant utilisation, le catalyseur à l'action de l'acide fluorhydrique dans les conditions de l'étape objet de la présente invention. Dans ce cas, on peut utiliser des précurseurs de fluorure plus facilement. Pour ce qui est des alliages ceux qui donnent les meilleurs résultats sont ceux qui présentent une corrosion après passage de l'acide fluorhydrique. Le plus souvent ladite espèce chimique est choisie parmi les fluorures, les oxyfluorures de chrome, d'aluminium, d'antimoine et de leurs mélanges.

Les dérivés à propriété catalytique peuvent être utilisés tels quels ou être supportés selon les techniques usuelles en matière de catalyse solide de réaction sur des gaz. Il va de soi que lorsque l'on désire utiliser comme catalyseur des composés qui sont des fluides à la températaure d'usage il est hautement préférable de les supporter.

En effet, selon la présente invention, il a pu être montré de manière surprenante que, en présence d'acide fluorhydrique, les catalyseurs ci-dessus notamment, à base d'espèces chimiques mentionnées ci-dessus permettaient d'obtenir un taux de conversion des halogénoformiates et des carbonates en fluorure correspondant, qui peuvent être très élevés, et dépasser 90 %.

En outre, ces catalyseurs n'ont que peu ou pas besoin d'être régénérés. Cette régénération peut être le cas échéant très aisément réalisée par passage sur le dit catalyseur d'un courant d'un gaz riche en oxygène, y compris l'oxygène à une température voisine de ou supérieure à la température d'utilisation.

Enfin il convient de mentionner que le temps de passage, ou temps de contact, défini par Tₚ = m/D (où m est la masse du catalyseur et D le débit gazeux à la température de réaction exprimée en cm³ par seconde) est en général compris entre 10⁻³ et 100 g x s/cm³, ce qui reste dans le domaine des cinétiques industrielles relativement rapides. Naturellement le temps de contact est spécifique de la texture et de la nature du catalyseur.

Un grand nombre de composés aromatiques fluorés peuvent être préparés de cette manière. Les limitations portent essentiellement d'une part sur la stabilité du noyau aromatique du substrat à la température choisie et d'autre part sur la tension de vapeur saturante de l'halogénoformiate ou du carbonate de départ. Les substituants donneurs sur les noyaux favorisent toutefois la réaction, cependant que les attracteurs ne la défavorisent que modéremment.

A la température de mise en oeuvre du procédé, ces derniers composés doivent avantageusement avoir une tension de vapeur saturante au moins agale à 10⁻³ de préférence à 10⁻² MPa.

Par ailleurs, pour obtenir des rendements importants, la cinétique de décomposition du fluorure formé doit être au plus égale à celle de sa formation.

Lorsque la vitesse de décomposition, bien que répondant à la contrainte ci-dessus, est élevée, il est préférable de faire une trempe du mélange réactionnel immédiatement après le contact avec le catalyseur.

Avantageusement l'halogénoformiate ou le carbonate organique répond à la formule (I) suivante :

(R₁)ₙ - Ar - O - CY - A (I)

avec Y défini comme précédemment, de préférence oxygène,
dans laquelle :
- Ar représente un radical aromatique monocyclique hétérocyclique ou non, polycyclique hétérocyclique ou non, avantageusement condensé ;
- Les radicaux R₁, semblables ou différents, représentent :
   - un motif electro attracteur tel que et en particulier halogène, nitro et/ou cyano
   - un groupe -Z-R₂,
      où Z peut être
      - une simple liaison ;
      - un atome d'oxygène ;
      - un atome de soufre ;
      - les groupes :
         - NR₃-;
         - CO-; -OCO-; -COO-;
         - SO-; -SO₂-; -SO₃-;
      où R₂ représente un atome d'hydrogène, un radical alcoyle, acyle ou aryle d'au plus 8 atomes de carbone,
      où R₃ représente un atome d'hydrogène, un radical alcoyle, acyle ou aryle d'au plus 8 atomes de carbone, ou forme avec R₂ et avec l'atome d'azote qui le porte, un hétérocycle azoté ;
- n représente le nombre de substituants et est égal à 0 ou à un entier au plus égal au nombre de positions substituables sur les noyaux aromatiques;
- A représente (R₁)n - Ar- O -, un halogène ou un groupe -OR'₂- dans lequel R_{'2} représente un radical aliphatique contenant 1 à 4 atomes de carbone éventuellement halogéné ou un radical aromatique (éventuellement substitué par un groupe alkyle contenant 1 à 4 atomes de carbone, alcoxy contenant 1 à 4 atomes de carbone, alkylthio contenant 1 à 4 atomes de carbone, nitro, halogéno, hydroxy ) ;
les groupes R₁ vicinaux pouvant être reliés entre eux pour former un cycle hétero ou non.

Les groupes alcoyles (tel que défini dans le dictionnaire de la Chimie Duval Presses Scientifiques Internationnale PARIS VIe 1959) peuvent notamment être des restes aliphatiques linéaires, cycliques ou ramifiés d'au plus 6 atomes de carbone ou des restes arylaliphatiques ,ils peuvent être substitués au moins partiellement, en particulier par des halogènes, et comprennent les radicaux dérivés des perfluoroalcanes .

Le nombre de positions substituables est déterminable aisément à l'aide de règles simples connues de l'homme de métier.

Ainsi par exemple :
quand
Ar = phényle n ≤ 5
Ar = pyridyle ≤ 4
Ar = naphtyle ≤ 7
Ar = quinolyle ≤ 6
Il convient de signaler que la réaction est également applicable aux composés où Ar représente par exception un vinyle avec n ≤ 3.

Le nombre total de carbone du composé de formule I est avantageusement au plus égal à 50 de préférence à 30.
La réaction donne des résultats particulièrement bons lorsque dans la formule (I) Aᵣ représente un radical aromatique monocyclique et que n est au plus égal à 3.

On préfère encore plus parmi les composés de formule (I) ceux pour lesquels R₁ représente un groupe méthyle, un groupe nitro ou encore ceux pour lesquels R₁ représente un groupe halogène et n est égal à 1.

On préfère également parmi les composés de formule (I) ceux pour lesquels A représente un halogène choisi parmi le fluor, le chlore ou le brome, ou groupe -OR'₂ dans lequel R'₂ représente un groupe de formule -O-Ar-(R₁)ₙ. Plus spécifiquement, le fluor est moins réactif et le brome plus réactif ; le meilleur compromis réactivité-coût est le chlore, le brome étant réservé aux synthèses les plus difficiles.

Avec le Bromoformiate, il est préférable d'utiliser un rapport acide fluorhydrique/halogénoformiate dans la zone élevée des fourchettes spécifiées ci-dessus.
Un des avantages du procédé est de pouvoir traiter des mélanges réactionnels comprenant à la fois carbonate et halogénoformiate.
Les matières premières préférées dans le cadre de la présente invention sont :
- le fluoroformiate de phényle (ce dernier pouvant être substitué)
- le chloroformiate de phényle (ce dernier pouvant être substitué)
- le carbonate de diphényle avantageusement symetrique (les phényles pouvant être substitués)

On ne s'écartera pas de l'invention en fabriquant in situ carbonates ou halogénoformiates par action d'halogénure de l'acide carbonique (par exemple phosgène) ou de leurs dérivés (par exemple polyphosgène) sur les phénols correspondants.

On ne s'écartera pas non plus de l'invention en réalisant des difluorures, voire des polyfluorures à partir d'halogénoformiate ou de carbonate issus de di ou de polyphénols.

Malgré le caratère très spécifique de l'atome de fluor et du fluorure, il a pu être montré, notamment par des essais semi-quantitatifs, que l'on pouvait synthetiser de la même manière d'autres halogénures et en particulier des chlorures en remplaçant le gaz fluorhydrique par l'acide gazeux halohydrique correspondant et dans le catalyseur les fluorures par les halogènures correspondants.

Cette simple modification, toute chose étant égale par ailleurs, permet l'obtention de l'halogènure désiré. Toutefois il est preférable d'utiliser des halogénoformiates ou équivalents qui présentent sur le carbone carbonique l'halogénure désiré ou un halogénure dont le nombre atomique est plus élevé. Dans le cas contraire il convient de se placer dans la zone haute des rapports acide halohydrique /substrat.

Les exemples non limitatifs suivants illustrent l'invention.

### EXEMPLES 1 à 2 : CATALYSEUR OXYDE DE CHROME

### Exemple 1

Un courant de chloroformiate de phényle(0,21 mmole/mn) vaporisé, est mélangé avec de l'acide fluorhydrique gazeux, à une température de 300°C, pour donner un milieu réactionnel contenant 48 moles d'Acide fluorhydrique par mole de chloroformiate de phényle.
Ce mélange passe au travers d'un réacteur tubulaire en Hastelloy C 276 de 35 cm de long et 2,0 cm de diamètre interne rempli de catalyseur au chrome (pastilles) préparé selon M. GARNIER Bull.Soc.Chim.Fr.1984 n°3-4,Page 91 à 96.
La température du lit catalytique est maintenue à 400°C et le temps de passage est de 2g.s/cm3.
Les produits gazeux sont condensés, dilués dans l'eau, neutralisés par la soude ( NaOH) aqueuse, extraits par le dichlorométhane.
La solution dans le dichlorométhane est analysée par chromatographie en phase vapeur, résonance magnétique nucléaire et spectrométrie de masse.
Le rendement en fluorobenzène est de 70 %

### Exemple 2

La procédure de l'exemple 1 est répétée sauf que l'on remplace le catalyseur au chrome par un catalyseur à l'oxyde de chrome sur alumine (HARSHAW (R 0101T 1/8)) traité préalablement à l'acide fluorhydrique. Le rapport molaire HF/chloroformiate est de 100, et le temps de contact de 0,4 g.s/cm3.
Le rendement en fluorobenzène est de 50 %.

### EXEMPLES 3 à 5 CATALYSEUR METALLIQUE

### Exemple 3

Un courant de chloroformiate de phényle (0,25 mmole/mn) vaporisé est mélangé avec de l'acide fluorhydrique gazeux à une température de 400°C pour donner un milieu réactionnel contenant 47 moles d'acide fluorhydrique par mole de chloroformiate de phényle. Ce mélange est passé au travers d'un réacteur-tube en Hastelloy C276 de 35 cm de long et 2,0 cm de diamètre interne rempli de billes d'acier (diamètre 4mm).
La température du lit catalytique est maintenue à 400°C et le temps de passage (défini précédemment) est de 8,4 g.s/cm3.
Les produits gazeux de la réaction sont condensés, dilués dans l'eau, neutralisés par la soude aqueuse, extraits par le dichlorométhane. La solution dans le dichlorométhane est analysée par chromatographie en phase vapeur, résonance magnétique nucléaire, et spectrométrie de masse. Le rendement en fluorobenzène est de 81 %.

### Exemple 4

La procédure de l'exemple 1 est répétée en remplaçant le chloroformiate de phényle par le fluoroformiate de phényle, le rapport HF/fluoroformiate de phényle étant de 61 avec un temps de passage de 3,8 g.s/cm3.
Le rendement en fluorobenzène est alors de 15 %.

### Exemple 5

A titre de comparaison, la procédure de l'exemple 1 est répétée dans un réacteur rempli de billes d'Hastelloy C276 au lieu de billes d'acier. Le rendement en fluorobenzène est de 0,8 %.

### Exemple 6 CHLORO-1-FLUORO-4-BENZENE

Un courant de chloroformiate de chloro-4 phényle (0,22 mmoles/min) vaporisé, est mélangé avec de l'acide fluorhydrique gazeux, à une température de 307° C, pour donner un milieu réactionnel contenant 132 moles d'acide fluorhydrique par mole de chloro-4-chloroformiate de phényle.

Ce mélange passe au travers d'un réacteur tubulaire en Hastelloy C 276 de 35 cm de long et 2.0 cm de diamètre interne contenant 10 g de fluorure d'aluminium en billes.
La température du lit catalytique est maintenue à 307° C et le temps de passage tp est de 0.47g.s/cm3.

Le flux sortant du réacteur est collecté dans un pot de recette de 1 litre contenant 100 ml de dichlorométhane et 750 ml d'eau.

Le passage du mélange gazeux est arrêté après 71 min.

La recette est extraite par le dichlorométhane, et la phase organique lavée à l'eau, séchée-neutralisée sur fluorure de potassium, filtrée, analysée par chromatographie en phase vapeur, résonance magnétique nucléaire et spectrométrie de masse.

Le rendement en chloro1-fluoro-4-benzène est de 55 %.

### Exemple 7 FLUORO-3-TOLUENE

Un courant de méthyl-3-chloroformiate de phényle (0.43 mmoles/min) vaporisé, est mélangé avec de l'acide fluorhydrique gazeux, à une température de 307° C, pour donner un milieu réactionnel contenant 18 moles d'acide fluorhydrique par mole de méthyl-3-chloroformiate de phényle.

Ce mélange passe au travers d'un réacteur tubulaire en Hastelloy C 276 de 35 cm de long et 2.0 cm de diamètre interne contenant 10 g de fluorure d'aluminium en billes.

La température du lit catalytique est maintenue à 307° C et le temps de passage tp est de 0,8 g.s/cm3. Le flux sortant du réacteur est collecté dans un pot de recette de 1 litre contenant 100 ml de dichlorométhane et 750 ml d'eau.

Le passage du mélange gazeux est arrêté après 2 h 14 min.

La recette est extraite par le dichlorométhane, et la phase organique lavée à l'eau, séchée-neutralisée sur fluorure de potassium, filtrée, analysée par chromatographie en phase vapeur, résonance magnétique nucléaire et spectrométrie de masse.

Le rendement en fluoro-3-toluène est de 95 %.

### Exemple 8 FLUORO-1-NAPHTALENE

Un courant de chloroformiate de naphtyle-1 (0.23 mmoles/min) vaporisé, est mélangé avec de l'acide fluorhydrique gazeux, à une température de 409°C, pour donner un milieu réactionnel contenant 83 moles d'acide fluorhydrique par mole de chloroformiate de naphtyle-1.

Ce mélange passe au travers d'un réacteur tubulaire en Hastelloy C 276 de 35 cm de long et 2.0 cm de diamètre interne contenant 10 g de fluorure d'aluminium (billes de 2 mm de diamètre).

La température du lit catalytique est maintenue à 409° C et le temps de passage tp est de 0.6 g.s/cm3.

Le flux sortant du réacteur est collecté dans un pot de recette de 1 litre contenant 100 ml de dichlorométhane et 750 ml d'eau.
Le passage du mélange gazeux est arrêté après 72 min.
La recette est extraite par le dichlorométhane, et la phase organique est lavée à l'eau, séchée-neutralisée sur fluorure de potassium, filtrée et analysée par chromatographie en phase vapeur, résonance magnétique nucléaire et spectrométrie de masse.

Le rendement en fluoro-1-naphtalène est de 81 %.

### Exemple 9 DIFLUORO-1,4-BENZENE

Un courant de fluoro-4-chloroformiate de phényle (0.30 mmoles/min) vaporisé, est mélangé avec de l'acide fluorhydrique gazeux, à une température de 407° C, pour donner un milieu réactionnel contenant 94 moles d'acide fluorhydrique par mole de fluoro-4-chloroformiate de phényle.

Ce mélange passe au travers d'un réacteur tubulaire en Hastelloy C 276 de 35 cm de long et 2.0 cm de diamètre interne contenant 10 g de fluorure d'aluminium en billes.

La température du lit catalytique est maintenue à 407° C et le temps de passage tp est de 0.42 g.s/cm3.

Le flux sortant du réacteur est collecté dans un pot de recette de 1 litre contenant 100 ml de dichlorométhane et 750 ml d'eau.
Le passage du mélange gazeux est arrêté après 60 minutes.
La recette est extraite par le dichlorométhane, et la phase organique lavée à l'eau, séchée-neutralisée sur fluorure de potassium, filtrée, analysée par chromatographie en phase vapeur, résonance magnétique nucléaire et spectrométrie de masse.

Le rendement en difluoro-1,4-benzène est de 96 %.

### Exemple 10 FLUORO-4-METHOXY BENZENE

Un courant de méthoxy-4-chloroformiate de phényle (0.23 mmoles/min) vaporisé, est mélangé avec de l'acide fluorhydrique gazeux, à une température de 407° C, pour donner un milieu réactionnel contenant 40 moles d'acide fluorhydrique par mole de méthoxy-4-chloroformiate de phényle.

Ce mélange passe au travers d'un réacteur tubulaire en Hastelloy C 276 de 35 cm de long et 2.0 cm de diamètre interne contenant 10 g de fluorure d'aluminium en billes.

La température du lit catalytique est maintenue à 407° C et le temps de passage tp est de 1.25 g.s/cm3.

Le flux sortant du réacteur est collecté dans un pot de recette de 1 litre contenant 100 ml de dichlorométhane et 750 ml d'eau.

Le passage du mélange gazeux est arrêté après 2 h 24 minutes.

La recette est extraite par le dichlorométhane, et la phase organique lavée à l'eau, séchée-neutralisée sur fluorure de potassium, filtrée, analysée par chromatographie en phase vapeur, résonance magnétique nucléaire et spectrométrie de masse.

Le rendement en fluoro-4-méthoxybenzène est de 5 %, du fluoro-4-phénol est de 18 %.

### Exemple 11 FLUORO-2-BIPHENYLE

Un courant de phényle-2-chloroformiate de phényle (0.17 mmoles/min) vaporisé, est mélangé avec de l'acide fluorhydrique gazeux, à une température de 356° C, pour donner un milieu réactionnel contenant 224 moles d'acide fluorhydrique par mole de phényle-2-chloroformiate de phényle.

Ce mélange passe au travers d'un réacteur tubulaire en Hastelloy C 276 de 35 cm de long et 2.0 cm de diamètre interne contenant 10 g de fluorure d'aluminium en billes.

La température du lit catalytique est maintenue à 356° C et le temps de passage tp est de 0.42 g.s/cm3.

Le flux sortant du réacteur est collecté dans un pot de recette de 1 litre contenant 100 ml de dichlorométhane et 750 ml d'eau.

Le passage du mélange gazeux est arrêté après 60 minutes.

La recette est extraite par le dichlorométhane, et la phase organique lavée à l'eau, séchée-neutralisée sur fluorure de potassium, filtrée, analysée par chromatographie en phase vapeur, résonance magnétique nucléaire et spectrométrie de masse.

Le rendement en fluoro-2-biphényle est de 23 %.

### Exemple 12 BROMO-1-FLUORO-4-BENZENE

Un courant de bromo-4-chloroformiate de phényle (0.33 mmoles/min) vaporisé, est mélangé avec de l'acide fluorhydrique gazeux, à une température de 407° C, pour donner un milieu réactionnel contenant 76 moles d'acide fluorhydrique par mole de bromo-4-chloroformiate de phényle.

Ce mélange passe au travers d'un réacteur tubulaire en Hastelloy C 276 de 35 cm de long et 2.0 cm de diamètre interne contenant 10 g de fluorure d'aluminium en billes.

La température du lit catalytique est maintenue à 407° C et le temps de passage tp est de 0,4 g.s/cm3.

Le flux sortant du réacteur est collecté dans un pot de recette de 1 litre contenant 100 ml de dichlorométhane et 750 ml d'eau.

Le passage du mélange gazeux est arrêté après 50 minutes.

La recette est extraite par le dichlorométhane, et la phase organique lavée à l'eau, séchée-neutralisée sur fluorure de potassium, filtrée, analysée par chromatographie en phase vapeur, résonance magnétique nucléaire et spectrométrie de masse.

Le rendement en bromo-1-fluoro-4-benzène est de 70 %.

### Exemple 13 : FLUOROBENZENE SUR OXYDE DE TITANE

Un courant de chloroformiate de phényle (0.25 mmoles/min) vaporisé, est mélangé avec de l'acide fluorhydrique gazeux, à une température de 220°C, pour donner un milieu réactionnel contenant 152 moles d'acide fluorhydrique par mole de chloroformiate de phényle.

Ce mélange passe au travers d'un réacteur tubulaire en Hastelloy C 276 de 35 cm de long et 2.0 cm de diamètre interne contenant 10 g d'oxyde de titane préalablement fluoré par l'acide fluorhydrique à 220° C.

La température du lit catalytique est maintenue à 220° C et le temps de passage tp est de 0.44 g.s/cm3.

Le flux sortant du réacteur est collecté dans un pot de recette de 1 litre contenant 100 ml de dichlorométhane et 750 ml d'eau.

Le passage du mélange gazeux est arrêté après 50 minutes.

### Exemple 14 : Essais divers

D'autres essais ont été réalisé en utilisant le mode opératoire de l'exemple 1. Les résultats de ces essais sont résumés dans les tableaux suivants.

**TABLEAU I**

| CATALYSE PAR ALLIAGE | | | |
|---|---|---|---|
| Produit Départ | CATALYSEUR | TEMPERATURE | RENDEMENT PAR RAPPORT AU PRODUIT DE DEPART |
| ϕOCOCl | INOX | 290° C | 0,5 % |
| " | INOX | 400° C | 80 % |
| " | HASTELLOY C 276 | 400° C | 0,8 % |
| " | FER | 400° C | 0,5 % |
| " | HASTELLOY | 500° C | 2,5 % |
| ϕOCOF | INOX | 400° C | 26 % |

**TABLEAU II**

| CATALYSE PAR OXYDE METALLIQUE | | | |
|---|---|---|---|
| ϕOCOCl | CATA TFA | 300° C | 18 % |
| | Cᵣ₂ O₃ prolabo | 300° C | 7 % |
| " | Cᵣ₂ O₃ Al2O₃+M_{g}O | 300° C | 50 % |
| " | Cr Cl₃, 6H₂O | 500° C | 25 % |
| ϕOCOϕ | Al₂ O₃+Cr₂ O₃ MgO | 300° C | 15 % |

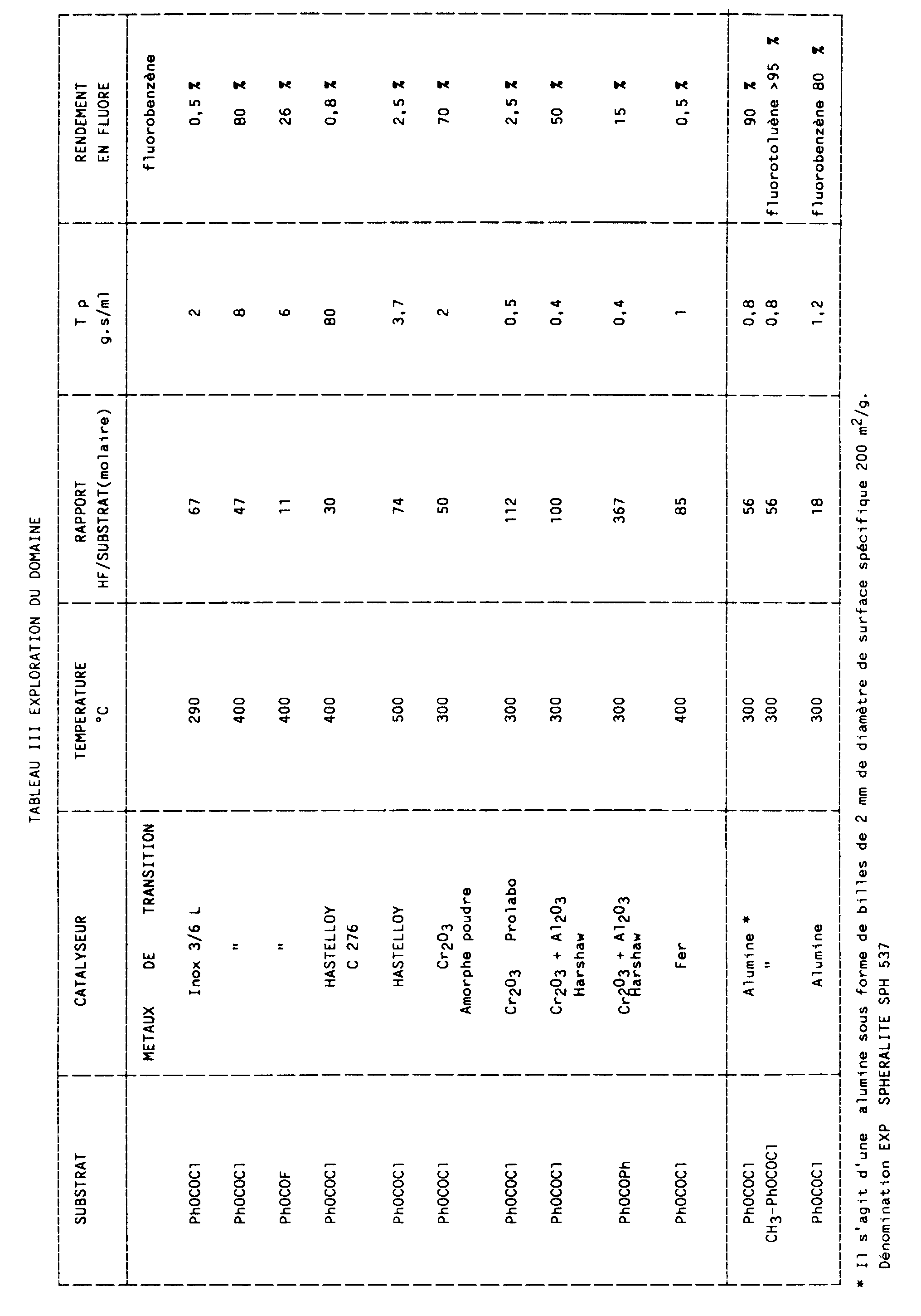

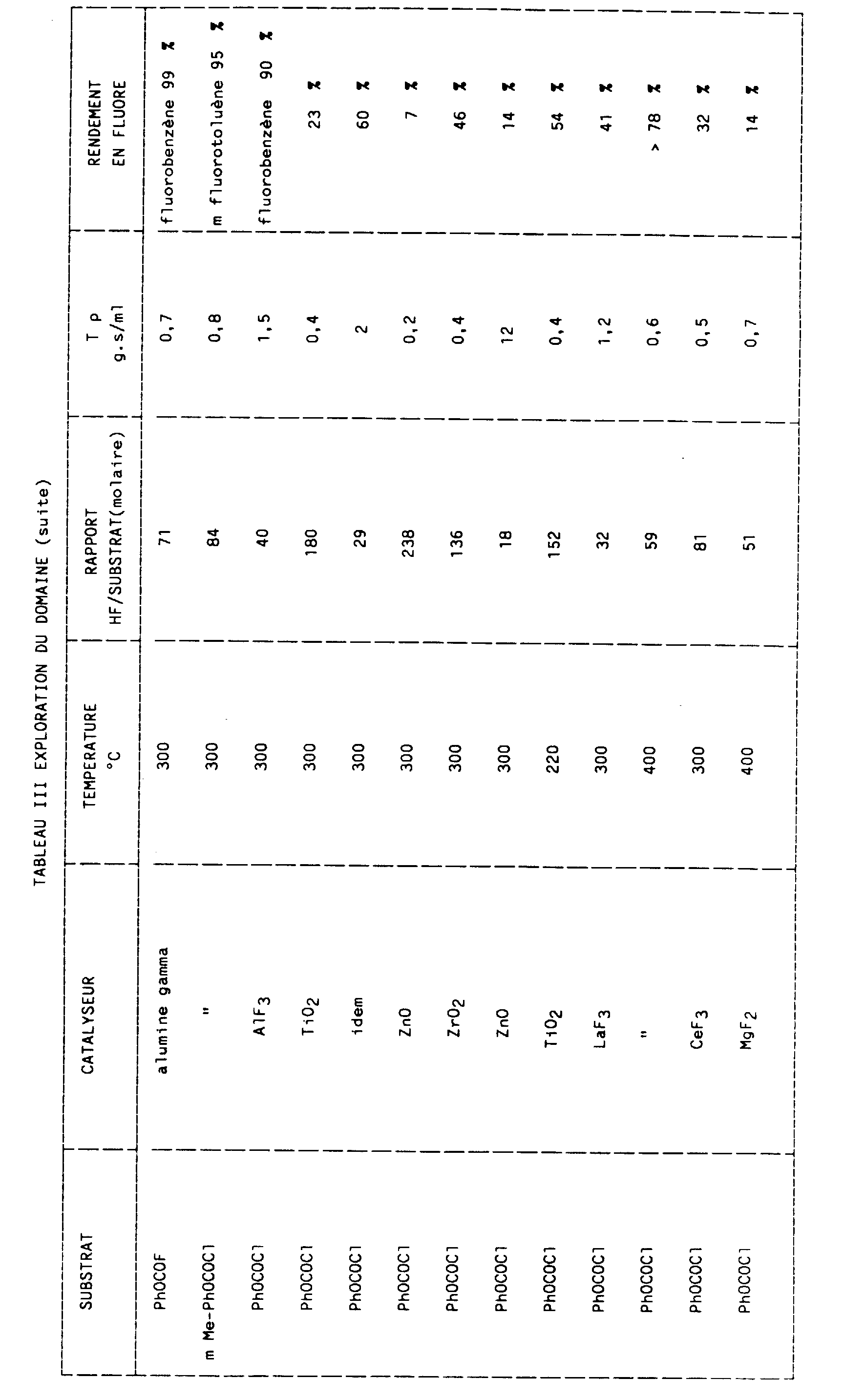

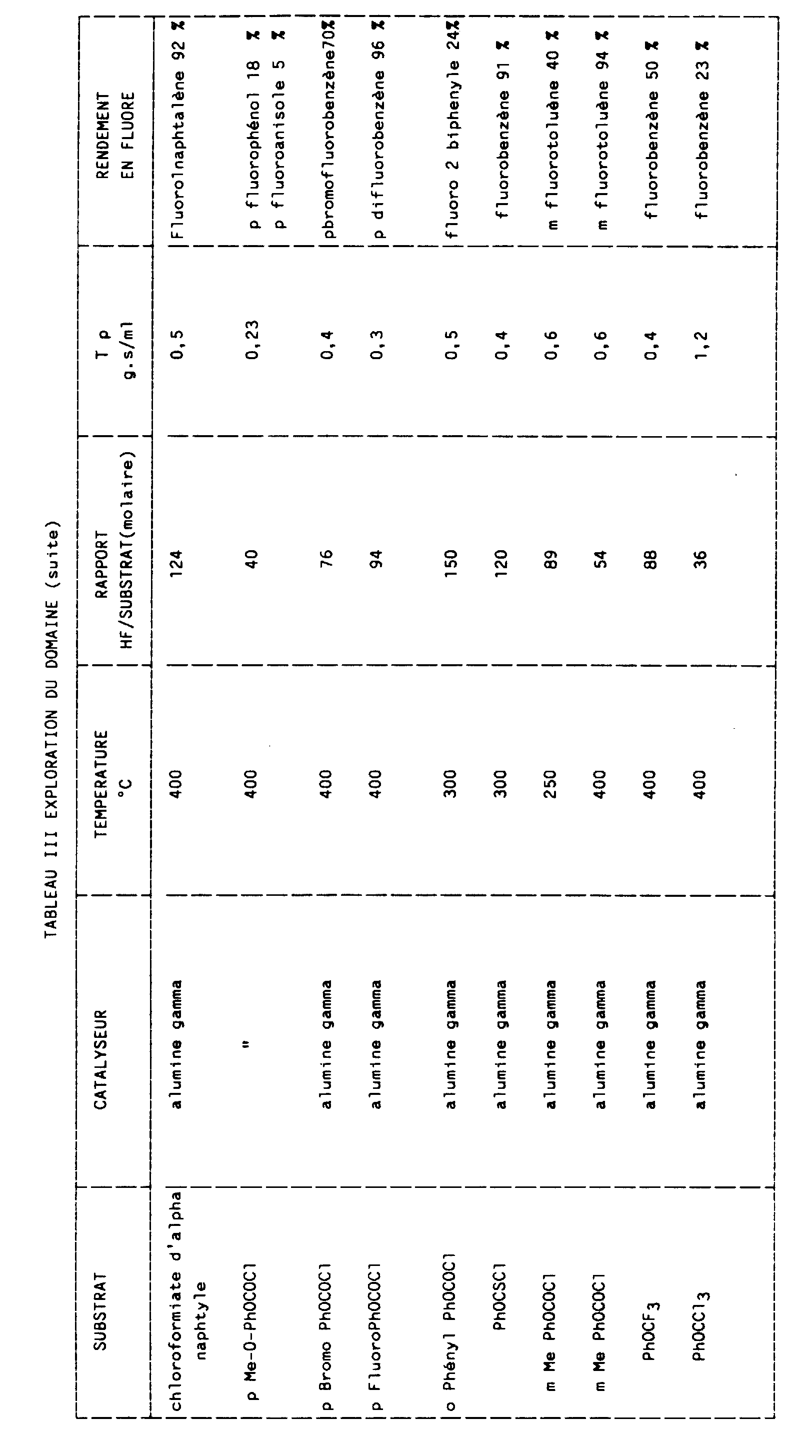

### EXEMPLE 15 : Essai comparatif : décomposition de Ph-O-CO-F sur alumine

Reproduction d'essai de la demande de brevet européen publiée sous le N° 0118241
le protocole des exemples précédents a été suivi avec les paramètres suivants :
température : 350°C
débit de gaz vecteur : 60 l/heure azote
débit de substrat : 50 millimole par heure (6ml/h)
temps de contact : 0,2 g.s/ml
les résultats sont rassemblés dans le tableau suivant :

| durée d'utilisation du catalyseur | 1h | 2h | 4h | 5h | 6h |
|---|---|---|---|---|---|
| rendement/produit de départ | 62% | 53% | 19% | 9% | 7% |

Ainsi le catalyseur est déjà très empoisonné au bout de 4 heures ; il y a production de goudron et de lourds. le catalyseur n'est pas régénérable.

### Exemple 16 : Importance et rôle de l'acide halohydrique.

le protocole de l'exemple précédent a été suivi pour divers types de catalyseur, sans acide halohydrique et avec les paramètres suivants :
température : 350°C
débit de gaz vecteur : 40 l/heure (azote)
temps de contact : 0,5 g.s/ml
les résultats sont rassemblés dans le tableau suivant :
(après une heure de passage pour assurer l'équilibre)

| catalyseur | rendement |
|---|---|
| CeF₃ | 3% |
| TiO₂ | 0% |
| ZrO₂ | 0% |
| Cr₂O₃ | 4% |

### Exemple 17 : essai de durée

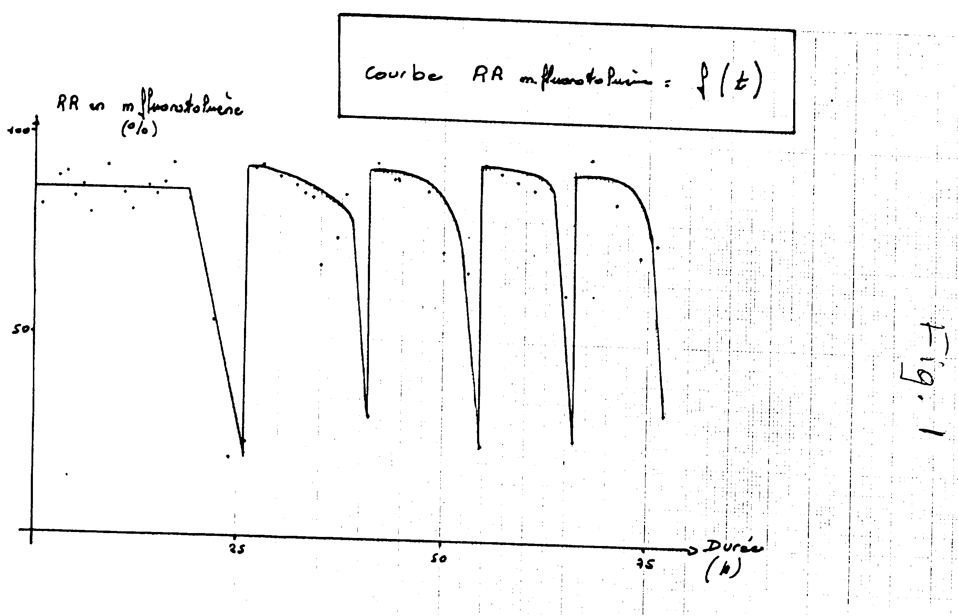

Le protocole général a été suivi avec les paramètres suivants :
- température :: 400°C
- temps de contact :: 0,5 g.s/ml
- rapport HF/Substrat :: 70
- substrat :: chloroformiate de meta cresol.
- catalyseur :: 10 g d'alumine gamma préalablement fluorée pendant deux heures par balayage dans les conditions de la réaction mais sans substrat. On a verifié qu'alors le sortant était identique à l'entrant.
la régénération est effectuée par passage d'air à 450°C pendant 3 heures avec un débit de 4 litres par heure.
les résultats sont rassemblés dans la courbe de la figure I .
les rendements sont des rendements par rapport au produit initial.

Les baisses brutales de rendement correspondent aux arrêts dus aux périodes de régénération (durées non figurées).

## Revendications

1. Procédé de préparation de fluorure de vinyl et de fluorure d'aryle caractérisé par le fait qu'il comporte une étape réactionnelle mise en contact d'un mélange gazeux contenant de l'acide fluorhydrique et un halogénoformiate ou un carbonate de vinyle et d'aryle, ou équivalents de formule générale:
-O-CY-A
où la valence libre est reliée audit vinyle ou aryle;
où Y représente un halcogène de préférence léger (S ou O) avantageusement oxygène ou bien deux halogènes de préférence semblables ;
où A représente, un halogène ou un groupe -OR'₂, voire un groupe -NR'₂R"₂ dans lequel R'₂ représente :
un radical aliphatique contenant 1 à 4 atomes de carbone éventuellement halogéné ; ou
un radical aromatique (éventuellement substitué par un groupe alkyle contenant 1 à 4 atomes de carbone, alcoxy contenant 1 à 4 atomes de carbone, nitro, halogéno, hydroxy) ;
dans lequel R"₂
représente un hydrogène ;
un radical aliphatique contenant 1 à 4 atomes de carbone éventuellement halogéné ou ;
un radical aromatique (éventuellement substitué par un groupe alkyle contenant 1 à 4 atomes de carbone, alcoxy contenant 1 à 4 atomes de carbone, alcoxy contenant 1 à 4 atomes de carbone, nitro, halogéno, hydroxy) avec un catalyseur à base d'acide(s) de Lewis.

2. Procédé selon la revendication 1 caractérisé par le fait que le rapport molaire entre l'acide fluorhydrique et l'halogénoformiate ou le carbonate de vinyle ou d'aryl est au moins égal à 1.

3. Procédé selon la revendication 2 caractérisé par le fait que ledit rapport molaire est compris entre 10 et 1 000.

4. Procédé selon l'une des revendications 1 à 3 caractérisé par le fait que l'halogénoformiate est du chloroformiate.

5. Procédé selon l'une des revendications 1 à 4 caractérisé par le fait que la température est comprise entre 200 et 800°C, de préférence entre 300 et 500°C.

6. Procédé selon l'une des revendications 1 à 5 caractérisé par le fait que le mélange gazeux est à une pression totale comprise entre 10⁻² et 20 MPa.

7. Procédé selon l'une des revendications 1 à 6 caractérisé par le fait que le mélange gazeux comporte en outre un gaz inerte vis-à-vis des espèces réactionnelles et du catalyseur.

8. Procédé selon l'une des revendications 1 à 7 caractérisé par le fait que ledit catalyseur à base d'acides de Lewis contient au moins une espèce chimique choisie dans le groupe constitué par les éléments de transition (éléments à sous couche d) les éléments métalliques des colonnes III b, IV b, V b et le silicium, sous forme de chalcogénure, d'halogénure, leurs mélanges et par leurs alliages.

9. Procédé selon les revendications 1 à 6 caractérisé par le fait que les éléments constitutifs des acides de Lewis sont choisis dans le groupe constitué par le chrome, les éléments des colonnes III a et b, le germanium et l'antimoine.

10. Procédé selon la revendication 8 caractérisé par le fait que les éléments à partir desquels les acides de Lewis sont constitués sont choisis parmi les éléments des colonnes III a et b.

11. Procédé selon les revendications 8 à 10 caractérisé par le fait que ladite espèce chimique est un alliage.

12. Procédé selon les revendications 8 à 10 caractérisé par le fait que ladite espèce chimique est choisie parmi les fluorures, les oxyfluorures de chrome, d'aluminium, d'antimoine et de leurs mélanges.

13. Procédé selon l'une des revendications 1 à 12 caractérisé par le fait que le temps de passage tp = m/D est égal entre 10⁻³ et 100 grammes/seconde par cm³.

14. Procédé selon l'une des revendications 1 à 13 caractérisé par le fait que l'halogénoformiate ou le carbonate organique répond à la formule (I) suivante :
(R1)n-Ar-O-CY-A (I)
dans laquelle :
- Y représente un chalcogène de préférence léger (S ou O) avantageusement oxygène ou bien deux halogènes de préférence semblables ;
- Ar représente un radical aromatique monocyclique hétérocyclique ou non, polycyclique hétérocyclique ou non, avantageusement condensé ;
- Les radicaux R₁, semblables ou différents, représentent :
- motif électro attracteur tel que et en particulier halogène, nitro et/ou cyano
- un groupe -Z-R₂,
où Z peut etre
- une simple liaison ;
- un atome d'oxygène ;
- un atome de soufre ;
- les groupes :
-NR₃- ;
-CO- ; -OCO- ; -COO- ;
-SO- ; -SO₂- ; -SO₃-,
où R₂ représente un atome d'hydrogène, un radical alcoyle, acyle ou aryle d'au plus 8 atomes de carbone,
où R₃ représente un atome d'hydrogène, un radical alcoyle, acyle ou aryle d'au plus 8 atomes de carbone, ou forme avec R₂ et avec l'atome d'azote qui le porte, un hétérocycle azoté ;
- n représente le nombre de substituants et est égal à 0 ou à un entier au plus égal au nombre de positions substituables sur les noyaux aromatiques ;
- A représente (R₁)n-Ar-O-, un halogène ou un groupe -OR'₂- dans lequel R'₂ représente un radical aliphatique contenant 1 à 4 atomes de carbone éventuellement halogéné ou un radical aromatique éventuellement substitué par un groupe alkyle contenant 1 à 4 atomes de carbone, alcoxy contenant 1 à 4 atomes de carbone, alkylthio contenant 1 à 4 atomes de carbone, nitro, halogéno, hydroxy) ;
les groupes R₁ vicinaux pouvant être reliés entre eux pour former un cycle hétéro ou non.

15. Procédé selon la revendication 2 caractérisé en ce que dans la formule (I) Ar représente un radical phényle et n est au plus égal à 5.

16. Utilisation comme catalyseur pour l'halogénation-décarboxylation d'un halogénoformiate ou d'un carbonate de vinyle et d'aryle, ou équivalent répondant à la formule générale -O-CY-A de la revendication 1, en présence d'acide halohydrique et en phase gazeuse, d'un composé contenant au moins une espèce chimique choisie dans le groupe constitué par les acides de Lewis notamment sous forme de chalcogénure, d'halogénure et par leurs mélanges.

## Patentansprüche

1. Verfahren zur Herstellung von Vinylfluorid und Arylfluorid, dadurch gekennzeichnet, daß es einen Verfahrensschritt umfaßt, bei dem eine gasförmige Mischung, enthaltend Fluorwasserstoff und ein Halogenformiat oder ein Vinyl- und Arylcarbonat, oder Äquivalente der allgemeinen Formel
-O-CY-A
mit einem Katalysator auf Basis einer oder mehrerer Lewis-Säuren in Kontakt gebracht werden,
wobei in der allgemeinen Formel die freie Bindung mit dem Vinyl oder Aryl verbunden ist;
wobei Y ein vorzugsweise leichtes Chalkogen (S oder O), vorzugsweise Sauerstoff, oder auch zwei Halogene, vorzugsweise die gleichen, darstellt;
wobei A darstellt: ein Halogen oder eine Gruppe -OR'₂ oder sogar eine Gruppe -NR'₂R"₂, wobei R'₂ darstellt:
einen gegebenenfalls halogenierten aliphatischen Rest mit 1 bis 4 Kohlenstoffatomen; oder
einen aromatischen Rest (der gegebenenfalls substituiert ist mit einer Alkylgruppe mit 1 bis 4 Kohlenstoffatomen, mit einer Alkoxygruppe mit 1 bis 4 Kohlenstoffatomen, mit einer Nitrogruppe, mit einem Halogen oder einer Hydroxygruppe);
wobei R"₂ darstellt:
Wasserstoff;
einen gegebenenfalls halogenierten aliphatischen Rest mit 1 bis 4 Kohlenstoffatomen; oder
einen aromatischen Rest (der gegebenenfalls substituiert ist mit einer Alkylgruppe mit 1 bis 4 Kohlenstoffatomen, mit einer Alkoxygruppe mit 1 bis 4 Kohlenstoffatomen, mit einer Nitrogruppe, mit einer Halogen- oder mit einer Hydroxygruppe).

2. Verfahren nach Anspruch 1, dadurch gekennzeichnet, daß das Molverhältnis zwischen Fluorwasserstoff und Halogenformiat oder Vinyl- oder Arylcarbonat mindestens gleich 1 ist.

3. Verfahren nach Anspruch 2, dadurch gekennzeichnet, daß das Molverhältnis zwischen 10 und 1.000 liegt.

4. Verfahren nach einem der Ansprüche 1 bis 3, dadurch gekennzeichnet, daß das Halogenformiat Chlorformiat ist.

5. Verfahren nach einem der Ansprüche 1 bis 4, dadurch gekennzeichnet, daß die Temperatur zwischen 200 und 800 °C liegt, vorzugsweise zwischen 300 und 500 °C.

6. Verfahren nach einem der Ansprüche 1 bis 5, dadurch gekennzeichnet, daß die gasförmige Mischung bei einem Gesamtdruck zwischen 10⁻² und 20 MPa vorliegt.

7. Verfahren nach einem der Ansprüche 1 bis 6, dadurch gekennzeichnet, daß die gasförmige Mischung außerdem ein Gas umfaßt, das gegenüber den Reaktionsspezies und dem Katalysator inert ist.

8. Verfahren nach einem der Ansprüche 1 bis 7, dadurch gekennzeichnet, daß der Katalysator auf Basis der Lewis-Säuren mindestens eine chemische Spezies enthält, die ausgewählt ist aus der Gruppe von Übergangsmetallelementen (Elemente mit einer d-Unterschicht), Metallelementen der Gruppen IIIb, IVb, Vb und Silicium, und zwar in Form von Chalkogeniden und Halogeniden, aus deren Mischungen und deren Gemengen.

9. Verfahren nach den Ansprüchen 1 bis 6, dadurch gekennzeichnet, daß die Elemente, aus denen die Lewis-Säuren bestehen, ausgewählt sind aus der Gruppe von Chrom, Elementen der Gruppen IIIa und b, Germanium und Antimon.

10. Verfahren nach Anspruch 8, dadurch gekennzeichnet, daß die Elemente, aus denen die Lewis-Säuren bestehen, ausgewählt sind aus den Elementen der Gruppen IIIa und b.

11. Verfahren nach den Ansprüchen 8 bis 10, dadurch gekennzeichnet, daß die chemische Spezies ein Gemenge ist.

12. Verfahren nach den Ansprüchen 8 bis 10, dadurch gekennzeichnet, daß die chemische Spezies ausgewählt ist aus Fluoriden und Oxyfluoriden des Chroms, des Aluminiums und des Antimons sowie deren Mischungen.

13. Verfahren nach einem der Ansprüche 1 bis 12, dadurch gekennzeichnet, daß die Durchtrittszeit tp=m/D zwischen 10⁻³ und 100 g/Sekunde pro cm³ liegt.

14. Verfahren nach einem der Ansprüche 1 bis 13, dadurch gekennzeichnet, daß das Halogenformiat oder das organische Carbonat der folgenden Formel (I) entspricht
(R₁)ₙ-Ar-O-CY-A (I)
in der:
- Y ein vorzugsweises leichtes Chalkogen (S oder O), vorzugsweise Sauerstoff, oder auch zwei Halogene, vorzugsweise die gleichen, darstellt;
- Ar einen homo- oder heterocyclischen monocyclischen oder hetero- oder homocyclischen polycyclischen, vorzugsweise kondensierten, aromatischen Rest darstellt;
- die Reste R₁, gleich oder verschieden, darstellen;
- eine elektronenziehende Einheit, wie z.B. insbesondere Halogen, Nitro und/oder Cyano,
- eine Gruppe -Z-R₂, wobei Z sein kann:
- eine Einfachbindung;
- ein Sauerstoffatom;
- ein Schwefelatom;
- die Gruppen:
-NR₃-;
-CO-; -OCO-; -COO-;
-SO-; -SO₂-; -SO₃-;
wobei R₂ ein Wasserstoffatom, einen Alkylrest, einen Acylrest oder einen Arylrest mit höchstens 8 Kohlenstoffatomen darstellt,
wobei R₃ ein Wasserstoffatom, einen Alkylrest, einen Acylrest oder einen Arylrest mit höchstens 8 Kohlenstoffatomen darstellt oder mit R₂ und mit dem Stickstoffatom, das es trägt, einen stickstoffhaltigen Heterocyclus bildet;
- n die Anzahl der Substituenten darstellt und gleich 0 oder eine ganze Zahl ist, die höchstens so groß ist wie die Anzahl der substituierbaren Positionen in den aromatischen Ringen;
- A darstellt: (R₁)ₙ-Ar-O-, ein Halogen oder eine Gruppe -OR'₂-, in der R'₂ einen gegebenenfalls halogenierten aliphatischen Rest mit 1 bis 4 Kohlenstoffatomen oder einen gegebenenfalls mit einer Alkylgruppe mit 1 bis 4 Kohlenstoffatomen substituierten aromatischen Rest, einen Alkoxyrest mit 1 bis 4 Kohlenstoffatomen oder einen Alkylthiorest mit 1 bis 4 Kohlenstoffatomen, einen Nitrorest, ein Halogen oder eine Hydroxygruppe darstellt;
wobei die benachbarten Gruppen R₁ untereinander verbunden sein können, um einen Homo- oder Heterocyclus auszubilden.

15. Verfahren nach Anspruch 2, dadurch gekennzeichnet, daß in der Formel (I) Ar einen Phenylrest darstellt und n höchstens gleich 5 ist.

16. Verwendung eines Halogenformiats oder eines Vinyl- und Arylcarbonats oder eines der allgemeinen Formel-O-CY-A aus Anspruch 1 entsprechenden Äquivalents als Katalysator für die Halogenierung-Decarboxylierung in Gegenwart von Fluorwasserstoff und einer Verbindung, die mindestens eine chemische Spezies enthält, die ausgewählt ist aus der Gruppe von Lewis-Säuren, insbesondere in Form von Chalkogeniden und Halogeniden, und deren Mischungen, in der Gasphase.

## Claims

1. Process for the preparation of vinyl fluoride and aryl fluoride, characterized in that it comprises a reaction stage of bringing a gaseous mixture containing hydrofluoric acid and a vinyl or aryl haloformate or carbonate or equivalents of general formula:
-O-CY-A
where the free valency is linked to the said vinyl or aryl;
where Y represents a chalcogen, which is preferably light (S or O), advantageously oxygen, or alternatively two halogens, which are preferably identical;
where A represents a halogen or a group -OR'₂, or even a group -NR'₂R"₂ in which R'₂ represents:
an aliphatic radical containing 1 to 4 carbon atoms, which are optionally halogenated; or
an aromatic radical (optionally substituted with an alkyl group containing 1 to 4 carbon atoms, an alkoxy group containing 1 to 4 carbon atoms, or a nitro, halo or hydroxyl group);
in which R"₂
represents a hydrogen;
an aliphatic radical containing 1 to 4 carbon atoms, which are optionally halogenated; or
an aromatic radical (optionally substituted with an alkyl group containing 1 to 4 carbon atoms, an alkoxy group containing 1 to 4 carbon atoms, or a nitro, halo or hydroxyl group),
into contact with a catalyst based on Lewis acid(s).

2. Process according to Claim 1, characterized in that the molar ratio of hydrofluoric acid to the vinyl or aryl haloformate or carbonate is at least equal to 1.

3. Process according to Claim 2, characterized in that the said molar ratio is between 10 and 1000.

4. Process acording to one of Claims 1 to 3, characterized in that the haloformate is chloroformate.

5. Process according to one of Claims 1 to 4, characterized in that the temperature is between 200 and 800°C, preferably between 300 and 500°C.

6. Process according to one of Claims 1 to 5, characterized in that the gaseous mixture is at a total pressure of between 10⁻² and 20 MPa.

7. Process according to one of Claims 1 to 6, characterized in that the gaseous mixture additionally comprises a gas which is inert towards the reaction species and the catalyst.

8. Process according to one of Claims 1 to 7, characterized in that the said catalyst based on Lewis acids contains at least one chemical species chosen from the group consisting of the transition elements (elements containing a sublayer d), the metallic elements of columns III b, IV b, V b and silicon, in the form of chalcogenide, halide, mixtures thereof and of alloys thereof.

9. Process according to Claims 1 to 6, characterized in that the elements constituting the Lewis acids are chosen from the group consisting of chromium, the elements of columns III a and b, germanium and antimony.

10. Process according to Claim 8, characterized in that the elements constituting the Lewis acids are chosen from the elements of columns III a and b.

11. Process according to Claims 8 to 10, characterized in that the said chemical species is an alloy.

12. Process according to Claims 8 to 10, characterized in that the said chemical species is chosen from chromium, aluminium and antimony fluorides and oxyfluorides and mixtures thereof.

13. Process according to one of Claims 1 to 12, characterized in that the flow time tf = m/D is equal between 10⁻³ and 100 grams/second per cm³.

14. Process according to one of Claims 1 to 13, characterized in that the organic haloformate or carbonate corresponds to the following formula (I):
(R₁)ₙ-Ar-O-CY-A (I)
in which:
- Y represents a chalcogen, which is preferably light (S or O), advantageously oxygen, or alternatively two halogens, which are preferably identical;
- Ar represents a monocyclic, polycyclic, heterocyclic or otherwise, aromatic radical, advantageously condensed;
- The radicals R₁, which are identical or different, represent:
- electron-withdrawing unit such as, and in particular, halogen, nitro and/or cyano
- a group -Z-R₂,
where Z may be
- a single bond,
- an oxygen atom;
- a sulphur atom;
- the groups:
-NR₃-;
-CO-; -OCO-; -COO-;
-SO-; -SO₂-; SO₃-;
where R₂ represents a hydrogen atom or an alkyl, acyl or aryl radical containing not more than 8 carbon atoms, where R₃ represents a hydrogen atom, an alkyl, acyl or aryl radical containing not more than 8 carbon atoms, or forms, with R₂ and with the nitrogen atom which bears it, a nitrogenous heterocycle;
- n represents the number of substituents and is equal to 0 or to an integer not exceeding the number of positions capable of being substituted on the aromatic nuclei;
- A represents (R₁)ₙ-Ar-O-, a halogen or a group -OR'₂- in which R'₂ represents an aliphatic radical containing 1 to 4 carbon atoms, optionally halogenated, or an aromatic (optionally substituted by an alkyl group containing 1 to 4 carbon atoms), alkoxy containing 1 to 4 carbon atoms, alkylthio containing 1 to 4 carbon atoms, nitro, halo or hydroxyl radical;
it being possible for the vicinal groups R₁ to be linked together to form a ring which may or may not be a heterocycle.

15. Process according to Claim 2, characterized in that in formula (I) Ar represents a phenyl radical and n does not exceed 5.

16. Use as catalyst for the halogenation-decarboxylation of a vinyl and aryl haloformate or carbonate or equivalent corresponding to the general formula -O-CY-A of Claim 1, in the presence of halohydric acid and in gaseous phase, of a compound containing at least one chemical species chosen from the group consisting of Lewis acids, especially in the form of chalcogenide, halide and of mixtures thereof.
